# EUROPEAN PATENT APPLICATION

(11) **EP 4 015 640 A1**
(43) Date of publication of application: **22.06.2022**
(21) Application number: 20214592.6
(22) Date of filing: 16.12.2020
(51) Int. Cl.: C12P 19/02, C12N 1/14, C12N 9/24, C12N 9/42, C12N 15/63, C12P 21/00, C40B 50/04

(54) **PROCESS FOR THE PRODUCTION OF A FILAMENTOUS FUNGUS WHOLE BROTH ENZYME COMPOSITION WITH LOW VISCOSITY**

(71) Applicant: Clariant Produkte (Deutschland) GmbH, 65929 Frankfurt am Main (DE)
(72) Inventor: GAMAUF, Christian, 81241 München (DE); CLAREN, Jörg, 82131 Stockdorf (DE)
(74) Representative: Graser, Konstanze

(57) **Abstract**

The present invention relates to a process for the production of a filamentous fungus whole broth enzyme composition with low viscosity, a genetically modified filamentous fungus for production of the whole broth enzyme composition, the use of such a genetically modified filamentous fungus for the production of the filamentous fungus whole broth enzyme composition with low viscosity and a filamentous fungus whole broth enzyme composition produced by such a method.

SEQUENCE LISTING

<110> Clariant Produkte (Deutschland) GmbH

<120> PROCESS FOR THE PRODUCTION OF A FILAMENTOUS FUNGUS WHOLE BROTH ENZYME
COMPOSITION WITH LOW VISCOSITY

<130> 2020DE624-EP-EPA

<160> 8

<170> BiSSAP 1.3.6

<210> 1
<211> 2774
<212> DNA
<213> Trichoderma reesei

<220>
<223> native gene

<400> 1
atgatagcca caaggtatct gctgagaatc agcctgcttc tcgtgggctt atttgcgtca 60

catgccctat cgagcaccat caatgccgat caggagtgtg tttctgctgt ctttagtatt 120

gtgggaggtc tcagctttgt aggcgtcggc tatggcaagt actacgtagc caactgccag 180

aacccgctca aagttgcctc catctacgcc gtctccaaga cctactgccc gccctctgac 240

ctggatcctg gcatcgaata cgtcaagtcg ttatgtcgaa agtacggcgg cgcggaacct 300

ttatcggagg cagatgtggc tgccaacctg acggatgaag caatcagcca gtatcccatt 360

cttgatcaga ctgatgatct ggtctcgacg aatcttacaa caccggtttt gatctcgaag 420

cattggtttg atctgggctt caagacggag gtaagttttc ttggcctcat ttctcgttgt 480

gtaacaccgt tgctaagatt ctctaggtcg agtgggatgc tgcggctcga aatcgcgaaa 540

actatgggtg agattacccc ctctgtactg cttgaagaaa gcaaggagct gacatgtata 600

acactacaga tgggccatgt acggattctg gggcatcgtt ctcctcttcg gaacttttca 660

tcgcctcatc tcccacttcc tcgaaggccg tgtgcttccg tcgtcgaccg gccccgaaga 720

taccggatcg gctgatagcc gtgaaaaatc cagagcagga accaagtcac ttgggcatct 780

gtactcgaga atacgcaagg gccttactac tcccagtgtt ttccctccgt atcaccagca 840

gcttctgttt gggtgcacga tccctactcg gcttgagcta ttcatcgttg cctcgtactg 900

gatcgtcagc ttcgtgctct gctgcgtcag ctatcgtcca ttcgacggca acctatagta 960

agtagccata ctgaaaaaga tgaacaaact ccttacgaga tacaaacgac taacagaggc 1020

ttactggaag ctggcccaag gtttctgctc agctatggcg atatattgcc gaccgaacgg 1080

gcgtcatgtc gtacgccaac ctaccgctga tatggatgtt tagcggccga aataacatct 1140

tcatctggct gacgggctgg caattctcca cgttcaacct gttccacaga cacattgctc 1200

gcgtggcgac attgcaggcc attattcact ccgttggata cacggcattt tactacacgg 1260

ccggttacgg tatgttgatc gcttgcctct catggagagt ttgcattgaa gttctccgtt 1320

gacaagagta atttcagggt atcaattccc accattgttc cgtgagcctt ggttctacct 1380

cggcgttgtg gtaagccttt tggcagacat ttcttatggc ctgtttgctg atcatgaccc 1440

actgtaggca acgtgtgcga tgagctttat tgtcttcttc tccttcagct ggttccgcag 1500

aaagatttac gagtcattcc tactcatcca catcgtccta tctgtcgtgg tgattgtcgc 1560

cctgttctag tacgttctcc tcctggtccg gtgcttctca ttgtccatct catactgact 1620

agacttggaa tcacagccac acatccatct ttgacggcca atatgaccca tatctatggc 1680

ctctcgttgc catctggagc ttcgatcgtt tcctgagaat cgtccgggta gtctactgca 1740

atatccacgt tcggctgcgt aagacaaagc tccagagaag catcgcggcc atagcttacg 1800

accaggacgc caacatcatc accgtccaga tcaacacaca tgtgaaaccg ggaccaggac 1860

agcactatta tctgtaccag cccttccgcc tcaaaggctg ggagaatcac cctttcaccc 1920

tggcccattg gaaccagcct ggaggacaaa caggatcaac agatgtgcaa tcgagaaccg 1980

tggatggctc tgttgtgaca tcgttcgaga gcggttcgcc acatggaggc atggcaggcg 2040

agcatcttct ttccttctgg attcgcccct atgacggctg gactcgccat ctccgtgacg 2100

tctgtctcaa ggctcagacc aagtcccagg ttcccgcagt ccataaagag accatcctcc 2160

tcgaaggccc ctacggcatg gcggagccct tgtgggctta tgacgaggtc cttctcttcg 2220

ccggcgggtc tggtatcgcg accatggttc cgtacatact tgatcacatg gaaagaacca 2280

ctgcgtctaa caaagcgaga cgaacacgca cacgcgcatt gacgctcgtg tggtcaaatc 2340

gcagccaagc tttcatgaag cgggttgccc agcgcgagct cgccgcggca cttgcgcgag 2400

acgacatcga gtgtatgttt cactgcacaa gcactgcaaa ggagtcgctt gactcgctgc 2460

ccttgacgcc gaaggatgag atttcggcgg agaaggagtg taaaggatgg gcggagagca 2520

gtgtgctcag gggcaaggag gagacggatg tgtctgagtc gcctgatggt gcggctgtgg 2580

caattaggat gggcaggcca gatatcgggg tcatgattga gaaagcggct gagtctgcct 2640

ctgcgtctgg atcacggctg gcggtgtttg cgtgtggccc tggagagatg gcggatgcgg 2700

cgagggcggc gacatatcgg gcgatgaagc agagccattc tgtcgagtat ttcgaggagg 2760

cgtttgggtg gtaa 2774

<210> 2
<211> 5886
<212> DNA
<213> Artificial Sequence

<220>
<223> pSEQ1M

<400> 2
ctaaattgta agcgttaata ttttgttaaa attcgcgtta aatttttgtt aaatcagctc 60

attttttaac caataggccg aaatcggcaa aatcccttat aaatcaaaag aatagaccga 120

gatagggttg agtggccgct acagggcgct cccattcgcc attcaggctg cgcaactgtt 180

gggaagggcg tttcggtgcg ggcctcttcg ctattacgcc agctggcgaa agggggatgt 240

gctgcaaggc gattaagttg ggtaacgcca gggttttccc agtcacgacg ttgtaaaacg 300

acggccagtg agcgcgacgt aatacgactc actatagggc gaattggcgg aaggccgtca 360

aggccgcatg actgtttaaa cacactcctg atcggcattg atggtgctcg atagggcatg 420

tgacgcaaat aagcccacga gaagcaggct gattctcagc agataccttg tggctatcga 480

ggctggatga tcttcaggtc ctgggaagtc gactgtccca caaacagata agaactttcc 540

acggcacggg acgccagata tacatcagct cggcaacttg cgttatataa ctactcattc 600

ctacgatccc tcggcagctc gtgaaccgta caacggtgtc cgctcacgcc actgtgggct 660

tttcaaaaac caatacttct gcctgcaacc ggggaatctt gatcccactc agcccgtatc 720

catcaccact cggattccgt cccccaggta gttggcacgc ccttcatcaa cactcgcagg 780

atcaaggttc tatacccgca atataaagaa aaaaaacttc gtcaaggtgg aggcacgttg 840

ctcgacatgg gcttgatcag cggtctagat cagttgatgc atgtcctagg gtcgtatctg 900

actcaatcct ttaggtaaag gtggactgat cagaaatctt atcagattga tattgactca 960

tcagattaga ttagatctga tcctgccgtg cgagtggctc tgtcgaggag aaagcccact 1020

taatcttctc ccttgtcttg cctgattagg aacggtcctt tgtctcttct ccggcttcct 1080

tatgcgacat gccctccggg aataggctca agcagttgag ccatttaatt gcctatactg 1140

gttttttttg gcttgtgacg ctccggtctt ttctctcgga ttgtgtcgag atcaagattt 1200

tccagcaata tattggcaga tgtggattga ctggggagca actgactgcc gctgcaggcc 1260

ttgagatccc ggtttccgca aactttacat ccgaagttcc tcttcgaagt gaagacaaag 1320

agacccagat tttgcccaaa ggcccgatat tccagcagtc cgtattgcgg ctgagggtgg 1380

atcatccttg aagtggcgac agagcaagct tgtttttact cttctttatc tcgccatgga 1440

tagtccatcg ggatacggac actggtgctg cttcggagat atacttgttc agtctcacaa 1500

cgctacaatg ctatggctac tcaagtgttc cagagaggat tattgtcaat atgcgacttc 1560

tcctgatccc acaatgtttg gatcttcaca tattgatgct gatgatctgt gggctttcct 1620

tatagcctct attccactag atgtcctact atatcggtaa tctaggattt tacaggattt 1680

ctcagaaata ggtggctcag ccgtgactcg agactatgga tactacctac ttcaagacca 1740

actatgcacc aagagaggac agaataacat acctaagtaa gagccatagg aagtgggaaa 1800

cagccacagt actttgtact atctctagag caaatcagtc gccgtgcatc ctagtcaccg 1860

tataccactg cagactctaa tctctatctt aaccataagt atcctggtgt ttcacacagc 1920

aagggtagca gtacaaaagt accgtcacac aggccaagac ttggcatagt gggcagaggc 1980

agcggtccgg gtaagcagct ccgtcttcgg gtcatcgagt tagtaggcta tggccgggac 2040

atccatcgaa tgaagatacg aattgccaaa taggatctat gagagttgcc ataagccatc 2100

cgattataaa taagagatca ccagccgaga cgctaggctt aagaagagta gcctcgccag 2160

cagaatagca taggaatatg ttgtatcttt agtgcactag caaaaagagt catagcaact 2220

ggcgttgtag ttacactcca agtaagaata atggttctcg aatacatgta gccttgatga 2280

gactattctg gacgctgtta cccatgtgtg ccttgcatat atctcagtat gagagtttaa 2340

gatcccggca atgcagaagc gaagttgctc agcttcctaa caagacacag cccgggctct 2400

tgtctgttaa ctgcctgcat gcagaaaggg acgcacttct tcccacctac aagagactgg 2460

agaaatggag tgatatatca ctggacagac gagggaaaag agctcgactt cacgatcaaa 2520

gggcattacg actcacttcc ttcgctaatc agacgaaccc ctacttatta agggcaacat 2580

tcacactcta aacgattaat aacaccaaag tctgccatta tctacagatt atgctccatt 2640

cctagtatat ataaccatag gtagaggaga gctcggggtc ctgttaagca cttctcttaa 2700

gtatattatc tgctattttt aatagctagg ggatctaaat ctagggttat atacttctat 2760

ttctagtagt taactaaccc tatattaagg tagttactac tattttactt taattaaaca 2820

tgctactaat agtactagta aaactaggct aagtctattt tttactaagc ccttaaatat 2880

taccttaata atttcacccc cttttactta agactaggtt atttttaata ctagtactgc 2940

tactacttac tttatataag gtaaagttat atataatact acaccttccc tgcactttat 3000

ataatattct tatattactt attagcgctt attttctctc tttttataac tcttttatct 3060

tattaatgca tatccttaat aactataata actaatttta taataattac tactactacc 3120

tttatagcta taattataac tatatattaa aagctaagta aggatatata atatatttat 3180

atatattcta taccttacta cttacttata gtttaactac tttataaaac taaactatta 3240

aacttaatat ataaagctat aatatttttt ttaaatagta ttaaaactat tttaatactt 3300

ttctaatcct tattctttat tataagtaat atttatttaa ggtaactaac tttataaagt 3360

atataaaaag taaggaagaa tttaagtaac tttttaaaaa agagaataaa aaaaagctta 3420

agaaatacta taacttaata taaaaagtat atttaaagat aatttataac tataagatct 3480

ttttatataa aaatgctaag aatatagcag taaggatata ctatagtacc tttaagtctt 3540

ttatatactt ttttaaaggc actattttta actaggaagc taataaagtt aagagcttag 3600

cacttaatat aaatattcta gtttaaaaag gggataatat ttacttaagt tcttataagg 3660

actataggtt aatataatta cttttttatt aaaactgtaa ctaaaagcta aattattata 3720

gtaactgcag actataacta aatatttata agtattattt aataatataa cctaaggata 3780

ttaatattaa ccctatataa tatataaata ctgtttatta taattaataa ataaagccta 3840

tagttaatag tattataact agtaaagata agctaaagac agttacttta agaaagcagt 3900

tagtttaaac gactctgggc ctcatgggcc ttccgctcac tgcccgcttt ccagtcggga 3960

aacctgtcgt gccagctgca ttaacatggt catagctgtt tccttgcgta ttgggcgctc 4020

tccgcttcct cgctcactga ctcgctgcgc tcggtcgttc gggtaaagcc tggggtgcct 4080

aatgagcaaa aggccagcaa aaggccagga accgtaaaaa ggccgcgttg ctggcgtttt 4140

tccataggct ccgcccccct gacgagcatc acaaaaatcg acgctcaagt cagaggtggc 4200

gaaacccgac aggactataa agataccagg cgtttccccc tggaagctcc ctcgtgcgct 4260

ctcctgttcc gaccctgccg cttaccggat acctgtccgc ctttctccct tcgggaagcg 4320

tggcgctttc tcatagctca cgctgtaggt atctcagttc ggtgtaggtc gttcgctcca 4380

agctgggctg tgtgcacgaa ccccccgttc agcccgaccg ctgcgcctta tccggtaact 4440

atcgtcttga gtccaacccg gtaagacacg acttatcgcc actggcagca gccactggta 4500

acaggattag cagagcgagg tatgtaggcg gtgctacaga gttcttgaag tggtggccta 4560

actacggcta cactagaaga acagtatttg gtatctgcgc tctgctgaag ccagttacct 4620

tcggaaaaag agttggtagc tcttgatccg gcaaacaaac caccgctggt agcggtggtt 4680

tttttgtttg caagcagcag attacgcgca gaaaaaaagg atctcaagaa gatcctttga 4740

tcttttctac ggggtctgac gctcagtgga acgaaaactc acgttaaggg attttggtca 4800

tgagattatc aaaaaggatc ttcacctaga tccttttaaa ttaaaaatga agttttaaat 4860

caatctaaag tatatatgag taaacttggt ctgacagtta ccaatgctta atcagtgagg 4920

cacctatctc agcgatctgt ctatttcgtt catccatagt tgcctgactc cccgtcgtgt 4980

agataactac gatacgggag ggcttaccat ctggccccag tgctgcaatg ataccgcgag 5040

aaccacgctc accggctcca gatttatcag caataaacca gccagccgga agggccgagc 5100

gcagaagtgg tcctgcaact ttatccgcct ccatccagtc tattaattgt tgccgggaag 5160

ctagagtaag tagttcgcca gttaatagtt tgcgcaacgt tgttgccatt gctacaggca 5220

tcgtggtgtc acgctcgtcg tttggtatgg cttcattcag ctccggttcc caacgatcaa 5280

ggcgagttac atgatccccc atgttgtgca aaaaagcggt tagctccttc ggtcctccga 5340

tcgttgtcag aagtaagttg gccgcagtgt tatcactcat ggttatggca gcactgcata 5400

attctcttac tgtcatgcca tccgtaagat gcttttctgt gactggtgag tactcaacca 5460

agtcattctg agaatagtgt atgcggcgac cgagttgctc ttgcccggcg tcaatacggg 5520

ataataccgc gccacatagc agaactttaa aagtgctcat cattggaaaa cgttcttcgg 5580

ggcgaaaact ctcaaggatc ttaccgctgt tgagatccag ttcgatgtaa cccactcgtg 5640

cacccaactg atcttcagca tcttttactt tcaccagcgt ttctgggtga gcaaaaacag 5700

gaaggcaaaa tgccgcaaaa aagggaataa gggcgacacg gaaatgttga atactcatac 5760

tcttcctttt tcaatattat tgaagcattt atcagggtta ttgtctcatg agcggataca 5820

tatttgaatg tatttagaaa aataaacaaa taggggttcc gcgcacattt ccccgaaaag 5880

tgccac 5886

<210> 3
<211> 2388
<212> DNA
<213> Artificial Sequence

<220>
<223> Hygromycin B resistance marker

<400> 3
gttaacaaga cacagcccta taacttcgta taatgtatgc tatacgaagt tatataacgg 60

tgagactagc ggccggtccc cttatcccag ctgttccacg ttggcctgcc cctcagttag 120

cgctcaactc aatgcccctc actggcgagg cgagggcaag gatggagggg cagcatcgcc 180

tgagttggag caaagcggcc cggccgccat gggagcagcg aaccaacgga gggatgccgt 240

gctttgtcgt ggctgctgtg gccaatccgg gcccttggtt ggctcacaga gcgttgctgt 300

gagaccatga gctattattg ctaggtacag tatagagaga ggagagagag agagagagag 360

agagagggga aaaaaggtga ggttgaagtg agaaaaaaaa aaaaaaaaaa aaatccaacc 420

actgacggct gccggctctg ccacccccct ccctccaccc cagaccacct gcacactcag 480

cgcgcagcat cacctaatct tggctcgcct tcccgcagct caggttgttt tttttttctc 540

tctccctcgt cgaagccgcc cttgttccct tatttatttc cctctccatc cttgtctgcc 600

tttggtccat ctgccccttt gtctgcatct cttttgcacg catcgcctta tcgtcgtctc 660

ttttttcact cacgggagct tgacgaagac ctgactcgtg agcctcacct gctgatttct 720

ctccccccct cccgaccggc ttgacttttg tttctcctcc agtaccttat cgcgaagccg 780

gaagaacctc ttaacctcta gatgaaaaag cctgaactca ccgccacgtc tgtcgagaag 840

ttcctgatcg aaaagttcga cagcgtctcc gacctgatgc agctctcgga gggcgaagaa 900

tctcgtgctt tcagcttcga tgtaggaggg cgtggatatg tcctgcgggt aaatagctgc 960

gccgatggtt tctacaaaga tcgttatgtt tatcggcact ttgcatcggc cgcgctcccg 1020

attccggaag tgcttgacat tggggaattc agcgagagcc tgacctattg catctcccgc 1080

cgtgcacagg gtgtcacgtt gcaagacctg cctgaaaccg aactgcccgc tgttctgcag 1140

ccggtcgcgg aggccatgga tgcgatcgct gcggccgatc tcagccagac gagcgggttc 1200

ggcccattcg gaccgcaagg aatcggtcaa tacactacat ggcgtgattt catatgcgcg 1260

attgctgatc cccatgtgta tcactggcaa actgtgatgg acgacaccgt cagtgcgtcc 1320

gtcgcgcagg ctctcgatga gctgatgctt tgggccgagg actgccccga agtccggcac 1380

ctcgtgcacg cggatttcgg ctccaacaat gtcctgacgg acaatggccg cataacagcg 1440

gtcattgact ggagcgaggc gatgttcggg gattcccaat acgaggtcgc caacatcttc 1500

ttctggaggc cgtggttggc ttgtatggag cagcagacgc gctacttcga gcggaggcac 1560

ccggagcttg caggatcgcc gcggctccgg gcgtatatgc tccgcattgg tcttgaccaa 1620

ctctatcaga gcttggttga cggcaatttc gatgatgcag cttgggcgca gggtcgatgc 1680

gacgcaatcg tccgatccgg agccgggact gtcgggcgta cacaaatcgc ccgcagaagc 1740

gcggccgtct ggaccgatgg ctgtgtagaa gtactcgccg atagtggaaa ccgacgcccc 1800

agcactcgtc cgagggcaaa ggaatagatg catggctttc gtgaccgggc ttcaaacaat 1860

gatgtgcgat ggtgtggttc ccggttggcg gagtctttgt ctactttggt tgtctgtcgc 1920

aggtcggtag accgcaaatg agcaactgat ggattgttgc cagcgatact ataattcaca 1980

tggatggtct ttgtcgatca gtagctagtg agagagagag aacatctatc cacaatgtcg 2040

agtgtctatt agacatactc cgagaataaa gtcaactgtg tctgtgatct aaagatcgat 2100

tcggcagtcg agtagcgtat aacaactccg agtaccagcg aaagcacgtc gtgacaggag 2160

cagggctttg ccaactgcgc aaccttgctt gaatgaggat acacggggtg caacatggct 2220

gtactgatcc atcgcaacca aaatttctgt ttatagatca agctggtaga ttccaattac 2280

tccacctctt gcgcttctcc atgacatgta agtgcacgtg gaaaccatac ccaatataac 2340

ttcgtataat gtatgctata cgaagttata gggctcttgt ctgttaac 2388

<210> 4
<211> 21
<212> DNA
<213> Artificial Sequence

<220>
<223> SEQ1MHygRfw

<400> 4
aacaagacac agccctataa c 21

<210> 5
<211> 21
<212> DNA
<213> Artificial Sequence

<220>
<223> SEQ1MHygRrv

<400> 5

aacagacaag agccctataa c                                                21

<210> 6
<211> 18
<212> DNA
<213> Artificial Sequence

<220>
<223> SEQ1MKOfw

<400> 6
gagaccagat catcagtc                                                    18

<210> 7
<211> 20
<212> DNA
<213> Artificial Sequence

<220>
<223> SEQ1MKOrv

<400> 7
ctgtacctag caataatagc                                                  20

<210> 8
<211> 20
<212> DNA
<213> Artificial Sequence

<220>
<223> MSeq-01

<400> 8
ggttgcaggc agaagtattg                                                  20

## Description

The present invention relates to a process for the production of a filamentous fungus whole broth enzyme composition with low viscosity, a genetically modified filamentous fungus cell for production of the whole broth enzyme composition, the use of such a genetically modified filamentous fungus cell for the production of the filamentous fungus whole broth enzyme composition with low viscosity and a filamentous fungus whole broth enzyme composition produced by such a method.

Hydrolysate from lignocellulose-containing (or lignocellulosic) biomass is coming more and more into focus as a valuable substrate for the production of various substances such as enzymes, lactic acid, fatty acids, iso-butanol, butanediol, succinic acid, itaconic acid but also ethanol. Ethanol originating from such a process is usually referred to as "bioethanol" or "biofuel". Production of the desired substances is often carried out by fermentation processes involving yeasts, bacteria or fungi capable of producing the desired end product.

One major drawback of such processes is the lignocellulosic biomass substrate itself. All biomasses usually referred to as "lignocellulosic substrate" contain a considerable amount of lignin (up to 40 wt.-%), cellulose (up to 55 wt.-%) and hemicelluloses (up to 55 wt.-%). Due to its origin (wood or weed plant-derived biomass) also the structure of the cell walls is significantly different from those of most other plant species which will influence hydrolysis of the substrate. Hydrolysis can be achieved by application of chemicals such as acids but is usually carried out by digestion of enzymes which will not contaminate the hydrolysate with e.g. salts resulting from chemical treatments.

The necessary hydrolytic breakdown of those polymers involves the use of several cellobiohydrolases, endoglucanases, β-glucosidases and oxidoreductases. To avoid costly supplementation of the single specific enzymes such hydrolytic breakdown is usually carried out by applying a so called "whole broth enzyme composition" produced by a microorganism, such as a filamentous fungus, capable of producing all enzymes necessary for hydrolysis of the substrate. The resulting glucose and cello-oligosaccharides can then easily be fermented to the desired end product - such as ethanol when using ordinary baker's yeast.

To attain economic feasibility, a high yield of the desired end product but also high yield of the produced whole broth enzyme composition is a necessity. This applies in particular when the end product is bioethanol which has to compete with ordinary and cheap mineral-oil derived fuel products on the market. Producing monomeric sugars from cellulose and hemicellulose at high yields is far more difficult than deriving sugars from sugar- or starch-containing crops, e.g. sugarcane or maize (Van Dyck and Pletschke, 2012). Therefore, although the cost of lignocellulosic biomass is far lower than that of sugar and starch crops, the cost of obtaining sugars from such materials for fermentation into e.g. bioethanol has often been considered to be too high to be industrially feasible. For this reason, it is crucial to solve the problems involved in the conversion of lignocellulosic biomass to hydrolysate.

One problem is the high viscosity of the fermentation broth of the fungus, especially of a filamentous fungus, which is needed for production of the whole broth enzyme composition. In order to obtain a high yield of enzymes, a strong growth of the fungus is desired, however, a strong growth comes with a high content of fungus biomass within the fermentation broth. Fungi, which are known to consist of i.a. hyphae are known within the art as rendering any fermentation substrate into a high-viscous composition. This effect is significantly more distinct when a filamentous fungus is used which exhibits a sponge-like, slimy appearance.

High viscosity causes many problems, as the fungus needs constant oxygen supply by aeration during growth. In addition, cooling of the fermenter, especially in industrial-scale production is required. Both can only be guaranteed by constant stirring - on the one hand to distribute the air bubbles homogenously within the broth, and on the other hand to facilitate constant heat-exchange with the cooling devices. The higher the viscosity of the broth the more energy needs to be spent to realize effective stirring within the reactor. Further, more air as to be pressed into the reactor causing also higher energy consumption within the compressor and sterile-filter unit. Thus, both CAPEX and OPEX increase with increasing viscosity of the fermentation broth. An alternative measure - less cell mass production - is also not attractive for commercial production as this would always be accompanied by a lower yield of whole broth enzyme production.

As lignocellulosic biomass is a substrate which already provides several challenges any further cost increase has to be avoided when applying such processes to commercial scale production.

The inventors of the present invention have therefore set themselves the task to develop a process for the production of filamentous fungus whole broth enzyme composition with low viscosity while maintaining a high yield of enzymes within the broth.

The task has been solved by a process for production of a whole broth enzyme composition, comprising the following steps:
(a) providing a fermentation medium, originating from hydrolysis of lignocellulosic biomass, with a glucose content of from 5 to 450 g/L, a xylose content of from 2 to 300 g/L, a density of from 1 to 2 kg/L and a dry matter content of from 10 to 75 wt.-%;
(b) addition of at least one filamentous fungus cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a whole broth enzyme composition.

Within the present invention the term "whole broth" is to be understood to consist of or to contain a partly or completely fermented medium and may even contain components of the original medium but also any compound generated during the fermentation process. "Whole broth" may also contain part of or all of the microbial biomass of the fermentation microorganism.

Within the present invention the term "whole broth enzyme composition" is to be understood as any whole broth as defined herein containing at least one enzyme. The at least one enzyme may have been added to the whole broth, may have been part of the original fermentation medium but may also be generated during the production process according to the present invention. "Whole broth enzyme composition" may also contain a mixture of two or more of such enzymes.

Within the present invention the whole broth enzyme composition preferably contains at least one enzyme belonging to the class of hydrolases. Within a particularly preferred embodiment of the present invention, the whole broth enzyme composition contains at least one enzyme belonging to the class of hydrolases which has been produced by the at least one filamentous fungus cell. Within another also particularly preferred embodiment, the whole broth enzyme composition contains at least one enzyme belonging to the class of cellulases and at least one enzyme belonging to the class of hemicellulases which has been produced by the at least one filamentous fungus cell.

Within the present invention, the term "enzyme belonging to the class of hydrolases" is to be understood as comprising any enzyme, capable of the hydrolysis of a chemical bond. Enzymes belonging to the class of hydrolases are classified as EC 3 in the EC number classification of enzymes. According to the present invention, the term "hydrolases" comprises cellulases, hemicellulases and may also encompass pectinases, oxydases and accessory proteins.

As used within the present invention, the term "cellulase" refers to any enzyme capable of hydrolyzing cellulose polymers to shorter oligomers and/or glucose. Cellulases preferred within the whole broth enzyme composition include cellobiohydrolases (CBH) (EC 3.2.1.-), endo-1,4-β-glucanases (EG) (EC 3.2.1.4).), beta-glucosidase (EC 3.2.1.4), cellobiose hydrolase (EC 3.2.1.21), glycoside hydrolase 61 (GH61 and CBM33).

As used within the present invention, the term "hemicellulase" refers to any enzyme capable of degrading or supporting the degradation of hemicellulose. Hemicellulases preferred within the whole broth enzyme composition include β-glucanases (EC 3.2.1.-), endo-xylanases (EC 3.2.1.8), β-xylosidases (EC 3.2.1.37), acetylxylan esterase (EC 3.1.1.72), acetylgalactan esterase (3.1.1.6), acetyl mannan esterase, feruloyl esterase (EC 3.1.1.73), glucuronoyl esterase (EC 3.1.1.-), α-L-arabinofuranosidase (EC 3.2.1.55), α-arabinopyranosidase (3.2.1.-), α-galactosidase (EC 3.2.1.22), β-galactosidase (EC 3.2.1.23), α-glucuronidases (EC 3.2.1.139), β-mannase (EC 3.2.1.78), β-mannosidases (EC 3.2.1.25), mannan 1,4-mannobiosidase (EC 3.2.1.100), arabinogalactan endo-beta-1,4-galactanase (EC 3.2.1.89), endo-beta-1,3-galactanase (EC 3.2.1.90), galactan endo-beta-1,3-galactanase (EC 3.2.1.181, glucuronoarabinoxylan endo-1,4-beta-xylanase (EC 3.2.1.136), alpha-L-fucosidase (EC 3.2.1.51), coniferin beta-glucosidase (EC 3.2.1.126), xyloglucan hydrolases (EC 3.2.1.150, 151, 155), xylan α-1,2-glucuronosidase (EC 3.2.1.131), endo-xylogalacturonan hydrolase (EC 3.2.1.-; GH28), α-amylase (EC 3.2.1.1), glucan 1,4-α-glucosidase (EC 3.2.1.3), galactan 1,3-galactosidase (GH43), -1,4,-endogalactanase (EC 3.5.1.89; GH53), α-rhamnosidase (EC 3.2.1.40), β-rhamnosidase (EC 3.2.1.43).

As used within the present invention, the term "pectinase" refers to any enzyme capable of degrading or supporting the degradation of pectin. Pectinases preferred within the whole broth enzyme composition include polygalacturonases (EC 3.2.1.15, 67, 82; GH28pectin methyl esterase (EC 3.1.1.11), pectin acetyl esterase (EC 3.1.1.-), rhamnogalacturonase (EC 3.2.1.-; GH28), rhamnogalacturonan acetylesterase (EC 3.1.1.86), rhamnogalacturonan galacturonohydrolase (EC 3.2.1.-), xylogalacturonan hydrolase (EC 3.2.1.-), pectin methylesterase (EC 3.1.1.11), beta-arabinofuranosidase (EC 3.2.1.55), beta-1 ,4-galactanase (EC 3.2.1.89), beta-1 ,3-galactanase (EC 3.2.1.90), beta-galactosidase (EC 3.2.1.23), alpha-galactosidase (EC 3.2.1.22), feruloyl acetyl esterase (EC 3.1.1.-), alpha-fucosidase (EC 3.2.1.51), (beta-fucosidase) (EC 3.2.1.38), beta-apiosidase (EC 3.2.1.-), alpha-rhamnosidase (EC 3.2.1.40), beta-rhamnosidase (EC 3.2.1.43), alpha-arabinopyranosidase (EC 3.2.1.-), beta-glucuronidase (EC 3.2.1.31), alpha-glucuronidase (EC 3.2.1.139), beta-xylosidase (EC 3.2.1.37) and alpha-xylosidase (EC 3.2.1.x).

As used within the present invention the term "accessory protein" refers to any enzyme capable of supporting cellulolytic enzyme activity. The term is well known to a person skilled in the art. Preferred accessory proteins within the whole broth enzyme composition include Expansin, Swollenin, Loosinin and CIP Proteins (EC 3.1.1.-; CE15).

As used within the present invention, the term "oxidative enzymes" refers to any enzyme capable of catalyzing an oxidation reaction. Oxidative enzymes preferred within the whole broth enzyme composition include lytic polysaccharide monooxygenase (LPMO) (AA9-11; previously GH61 and CBM33, resp.) (EC 1.14.99.53-56, 1.14.99.B10), lignin peroxidase (EC 1.11.1.14), manganese peroxidase (EC 1.11.1.13), aryl-alcohol oxidase (EC 1.1.3.7), glyoxal oxidase (EC 1.1.3.), carbohydrate oxidases (EC 1.1.3.4, 9, 10), cellobiose dehydrogenase (EC 1.1.99.18), catalase (hydrogen-peroxide oxidoreductase) (EC 1.11.1.6 or EC 1 .11.1.21), dye-decolorizing peroxidase (EC 1.11.1.19), laccase (EC 1.10.3.2), peroxidase (EC 1.11.1.x) and versatile peroxidase (EC 1.11.1.16).

The enzymes referenced within the present invention are classified according nomenclatures that are either based on the International Union of Biochemistry and Molecular Biology's Enzyme Nomenclature and Classification (http://www.chem.qmul.ac.uk/iubmb/enzyme/) or on Carbohydrate-Active EnZYmes (http://www.cazy.org/) database.

Within the present invention, the at least one enzyme belonging to the class of hydrolases amounts preferably to from 1 to 45 wt.-% (relative to the weight of the whole broth enzyme composition), further preferred to from 1 to 25 wt.-%, particularly preferred to from 1 to 20 wt.-%, also preferred to from 2 to 15 wt.-%, from 2 to 14 wt.-%, from 3 to 12 wt.-% and most preferred to from 5 to 11 wt.-%.

Within the present invention the term "fermentation medium originating from hydrolysis of lignocellulosic biomass" can be any medium which has been prepared by chemical, mechanical and/or enzymatic hydrolysis of lignocellulosic biomass material and preferably comprises prior mechanical and/or acidic pretreatment of the lignocellulosic biomass. Within a preferred embodiment of the inventive process, the hydrolysis has been carried out by mechanical and enzymatical hydrolysis or by sole enzymatic hydrolysis without the addition of any organic and/or inorganic acid(s). The hydrolysis of lignocellulosic biomass is known to a person skilled in the art, exemplary methods are for example described within Vishnu et al. 2012 (Trends in bioconversion of lignocellulose: Biofuels, platform chemicals & biorefinery concept in bioconversion of lignocellulose: Biofuels, platform chemicals & biorefinery concept. Progress in Energy and Combustion Science, August 2012, vol. 38 (4), 522-550) and Prasad et al. 2019 (Bioethanol production from waste lignocelluloses: A review on microbial degradation potential Chemosphere Volume 231, September 2019, p. 588-60).

Within the present invention the term "lignocellulose-containing biomass" is to be understood to comprise all kind of biomass known to a person skilled in the art as comprising lignocellulose. Particularly preferred lignocellulose-containing biomass according to the present invention include wood, cereal straw such as but not limited to wheat straw, rice straw, barley stray, rye straw and oat straw, and/or husks and/or brans thereof, bagasse, oat hulls, switch grass, cellulose, raw paper pulp (obtained from pulp and paper production) and mixtures thereof. Additional components may comprise one or more of the following components: purified cellulose, pulp, milk whey or molasses. Lignocellulosic biomass which is particularly suitable for hydrolysis according to the process of the present invention is selected from the group consisting of cereal straw, cereal bran, cereal husks, wood, bagasse and mixtures thereof.

In a preferred embodiment the lignocellulose-containing material contains at least 25 wt.-%, preferably at least 40 wt.-%, more preferably at least 70 wt.-%, even more preferably at least 80 wt.-% and most preferred at least 90 wt.-% lignocellulose. It is to be understood that the lignocellulose-containing material may also comprise other compounds such as proteinaceous material, starch, sugars, such as fermentable sugars and/or non-fermentable sugars.

Within the process of the present invention, the fermentation medium contains from 5 to 450 g/L glucose and from 2 to 300 g/L xylose, wherein glucose contents from 5 to 420 g/L, from 8 to 400 g/L and from 10 to 280 g/L are preferred and wherein xylose contents from 3 to 280 g/L, from ¹3 to 270 g/L and from 4 to 260 g/L are preferred. Further preferred ranges of glucose are from 10 to 450 g/L, from 40 to 400 g/L and from 50 to 350 g/L whereas further preferred ranges of xylose are from 10 to 280 g/L, from 30 to 250 g/L and from 50 to 220 g/L. Further preferred is a ratio from glucose to xylose selected from the range of from 5 to 1, such as a ratio selected from the range of from 3 to 1, from 4.0 to 1.5, of from 3.5 to 1.5 or of from 3.0 to 1.5. Ratios selected from the range of from 2.5 to 1.0 are most preferred as a maximum of glucose and xylose have been released from the lignocellulosic biomass during hydrolyzation enabling a higher yield of whole broth enzyme composition.

The fermentation medium originating from hydrolysis of lignocellulosic biomass has a high density of from 0.90 to 2.00 kg/L, preferably of from 0.95 to 1.90 kg/L, further preferred of from 1.00 to 1.50 kg/L and most preferred of from 1.05 to 1.35 kg/L.

The fermentation medium originating from hydrolysis of lignocellulosic biomass has a dry matter content of from 10 to 75 wt.-%, preferably of from 10 to 70 wt.-%, further preferred of from 20 to 65 wt.-%, from 30 to 65 wt.-% or from 40 to 60 wt.-% whereas a dry matter content of from 10 to 20 wt.-% and from 10 to 15 wt.-% is also preferred. The "providing" of the fermentation medium according to step (a) of the inventive process can be carried out by any method and within any means known to a person skilled in the art as suitable for the inventive process. Within a preferred embodiment the fermentation medium originating from hydrolysis of lignocellulosic biomass is provided within a batch or fed batch reactor which is preferred equipped with a stirring device and a cooling device.

Within a preferred embodiment of the inventive process, the fermentation medium has a furfural content of less than 0.5 g/L, preferably less than 0.2 g/L, further preferred less than 0.1 g/L, also preferred less than 0.05 g/L and is most preferred selected from the range of from 0.001 mg/L to 0.5 g/L or from 0.01 mg/L to 0.25 g/L.

Within another preferred embodiment of the inventive process, the fermentation medium has a hydroxymethyl furfural content of less than 0.5 g/L, preferably less than 0.2 g/L, further preferred less than 0.1 g/L, also preferred less than 0.05 g/L and is most preferred selected from the range of from 0.001 mg/L to 0.5 g/L or from 0.01 mg/L to 0.25 g/L.

Within another preferred embodiment from 0.05 to 5 wt.-% nitrogen are added during step (a) and/or (b) of the inventive process. Further preferred ranges are from 0.08 to 5 wt.-%, from 0.1 to 5 wt.-% and from 0.5 to 5 wt.-%. The nitrogen can be added in any form known to a person skilled in the art as suitable for the inventive purpose and may be added in form of ammonium sulfate, ammonia, urea or combinations thereof. The amount of nitrogen can be added by feeding or by adding the total amount to the fermentation medium at any time during step (a) and/or (b) of the inventive process. It is thereby preferred that the nitrogen is added as a 25% (wt.-/wt.) solution of ammonia or a 40 % (wt./wt.) solution of urea.

Within another preferred embodiment from 0.5 to 350 mg/L FeSO₄, MnSO₄, MgSO₄ and/or ZnSO₄ are added during step (a) and/or (b) of the inventive process. The amount of FeSO₄, MnSO₄, MgSO₄ and/or ZnSO₄ can be added by feeding or by adding the total amount to the fermentation medium at any time during step (a) and/or (b) of the inventive process. It is thereby preferred that FeSO₄ is added in an amount of from 0.5 to 35 mg/l and MgSO₄ is added in an amount of from 200 to 350 mg/l.

Within a particularly preferred embodiment of the inventive process no mono- and/or disaccharides, in particular no glucose, fructose or xylose, are added to the fermentation medium originating from hydrolysis of lignocellulosic biomass at any time during the inventive process.

Within a preferred embodiment of the inventive process the pH of the fermentation medium has been adjusted to a pH selected from the range of from pH 2.0 to pH 6.0, wherein ranges of from pH 3.0 to 5.5 and from pH 3.5 to 5.5 as well as from pH 3.5 to 5.0 are particularly preferred. The adjusting of the pH can be carried out by any means and method known to a person skilled in the art as suitable for the inventive purpose. Within the process of the present invention the pH is preferably adjusted by addition of an acid such as sulfuric acid or acetic acid, NaOH, H₃PO₄ or ammonia.

Within a preferred embodiment of the inventive process the process further comprises step (ai) concentration of the fermentation medium by evaporation, membrane filtration, thin layer evaporation, falling-film or downstream evaporation to decrease the weight of the fermentation medium by factor 2 to 6, wherein a decrease of weight of the fermentation medium by a factor of 2.5 to 6, 3 to 6, 3.5 to 6 and 4 to 6 is also preferred. The decrease of weight of the fermentation medium is thereby mostly achieved due to a decrease of water content of the original fermentation medium. It is thereby possible to achieve the decrease of weight of the fermentation medium by concentration of only part of the medium and blending the concentrated and non-concentrated medium before carrying out step (b) or by concentration of the complete fermentation medium to the desired end content, desired weight decrease, respectively.

Within a further preferred embodiment of the inventive process the fermentation medium originating from lignocellulosic biomass has a content of organic acids of less than 5 wt.-%, a content of inorganic acids of less than 6 wt.-%, a content of inorganic salts of less than 3 wt.-% and/or an arabinose content of less than 1 wt.-%, wherein the following contents are particularly preferred: 0.5 to 2.5 wt.-% organic acids, 0.5 to 5 wt.-% inorganic acids, 0.2 to 2.5 wt.-% inorganic salts and/or 0.05 to 0.75 wt.-% arabinose. It is thereby also particularly preferred that the content of water soluble chloride is below 0.5 wt.-%, the content of acetic acid is below 35 g/L and/or the content of Na-D/L-lactate is below 15 g/L.

According to step (b) of the inventive process, at least one filamentous fungus cell wherein SEQ ID NO: 1 has been disrupted is added to the fermentation medium. The addition can be carried out by any means and measure known to a person skilled in the art as suitable for the inventive process. Within a preferred embodiment, the at least one filamentous fungus cell is added in a quantity of from 10² to 10¹⁰ cells, preferably in a quantity of from 10³ to 10⁸ cells and most preferred in a quantity of from 10⁴ to 10⁷ cells per g of fermentation medium. The at least one filamentous fungus cell can thereby be added in dried form, as conidia or in form of a preculture, containing rest of preculturing medium. It is also possible to add the at least one filamentous fungus cell in form of a fully cultured medium (also referred to as main culture).

Within the present invention the term "filamentous fungus cell" is to be understood as any cell from any filamentous fungus existing in nature and/or known to a person skilled in the art. The term also comprises any filamentous fungus cell either of natural origin or modified. The term "modified" refers to genetically and non-genetically modified fungi. i.e. fungi which have been modified by genetic methods (e.g. transformation) and non-genetic methods e.g. chemical mutagenesis or irradiation, both of which are known to those skilled in the art. Within a preferred embodiment the at least one filamentous fungus cell is selected from the group consisting of *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes,* wherein *Trichoderma* and *Aspergillus* are particularly preferred, most preferred is *Trichoderma reesei* (teleomorph: *Hypocrea jecornia*). Within another preferred embodiment of the present invention, the at least one filamentous fungus cell is a genetically modified filamentous fungus cell with the with the ability to express at least one heterologous hydrolase enzyme, at least one heterologous pectinase enzyme, at least one heterologous oxidative enzyme and/or at least one heterologous accessory protein, preferably an enzyme belonging to the class of beta-glucosidases, to the class of xylanase enzymes, to the class of beta-xylosidase enzymes and/or to the class of lytic polysaccharide monooxygenase enzymes.

Within such a preferred embodiment, the at least one heterologous hydrolase enzyme preferably originates from another filamentous fungus such as - but not limited to *-Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes.* Within a particularly preferred embodiment the at least one filamentous fungus cell is a *Trichoderma reesei* cell and the at least one heterologous hydrolase enzyme originates from *Acremonium, Ajellomyces, Alternaria, Armillaria, Arthroderma, Aspergillus, Bionectria, Bipolaris, Ceriporiopsis, Chaetomium, Cladophialophora, Clohesyomyces, Colletotrichum, Coniochaeta, Coniosporium, Diaporthe, Dothistroma, Emericella, Epicoccum, Exophiala, Fomes, Fonsecaea, Fusarium, Gibberella, Grosmannia, Hebeloma, Hortaea, Humicola, Hypocrea, Hypoxylon, Irpex, Isaria, Kuraishia, Leucoagaricus, Madurella, Magnaporthe, Marssonina, Metarhizium, Moniliophthora, Myceliophthora, Mycosphaerella, Neurospora, Oidiodendron, Ophiostoma, Paecilomyces, Paraphaeosphaeria, Penicillium, Phanerochaete, Phialophora, Pleurotus, Pochonia, Pseudocercospora, Pseudogymnoascus, Pyrenophora, Rasamsonia, Rhinocladiella, Rhizopus, Rhizosphaera, Rhynchosporium, Setosphaeria, Sphaerulina, Sporothrix, Stachybotrys, Stemphylium, Talaromyces, Termitomyces, Tilletiaria, Torrubiella, Trametes, Trichoderma, Trichophyton, Uncinocarpus* and/or *Valsa* species.

According to the present invention, the at least one filamentous fungus cell as is a filamentous fungus cell wherein SEQ ID NO: 1 has been disrupted. The "disruption" can thereby be carried out by any means and measure known to the person skilled in the art as suitable for the purpose of disruption. The term "disruption" in particular comprises all techniques that either lead to the gene no longer being transcribed or to the protein encoded by the gene no longer being produced or only being produced in an inactive form.

Exemplary methods which can be used within the present invention are:
- the partial or complete removal from the genome of the gene, the region coding for the protein and/or the promoter or other regions necessary for the expression of the gene (= "deletion")
- the alteration of the DNA sequence of the coding region so that either the encoded protein is not produced at all, or only a shortened protein (= generation of a stop codon) or a protein with an altered amino acid sequence is produced which can no longer perform the function of the unchanged protein (= "mutation")
- the modification of the DNA sequence of the promoter or other regions necessary for the expression of the gene, so that the gene is no longer transcribed (= no RNA and therefore no protein is produced)
- the expression of RNA with a sequence complementary to that of the target gene. This leads to hybridization (= pairing of complementary sequences) of the two RNAs and to a degradation of this double-stranded RNA. As a result, no RNA of the target gene is available for protein synthesis (= RNA interference).

Within the present invention SEQ ID NO:1 is defined within the sequence protocol.

Mixing according to step (c) of the process of the present invention is carried out for a time period from 1 minute to 10 days, preferably from 10 hours to 7 days, further preferred from 24 hours to 5 days, preferably under constant stirring with a power input from 150 to 20000 W/ m³ and more preferably from 500 to 15000 W/m³ and under oxygen controlled conditions. The average dissolved oxygen level is preferably selected from 0.01% to 80%, preferred from 0.1% to 50%, particularly preferred from 5% to 30% and most preferred from 12% to 28%. Within a particularly preferred embodiment, the dissolved oxygen level is controlled by a stirrer or compressed air flow or internal reactor pressure or a combination of two or three of these measures. Furthermore, mixing according to step (c) of the inventive process is carried out at a temperature of from 20 to 35 °C, preferably at a temperature of from 21 to 34 °C wherein a temperature selected from the range of from 22 to 33 °C is also preferred.

"Mixing" according to step (c) of the process of the present invention is preferably conducted in a batch mode (discontinuous), in the fed-batch mode or in a continuous mode. Most preferably, the inventive process is conducted in the batch mode.

"Obtaining" according to step (d) of the inventive process is preferably carried out by harvesting the whole fermentation broth at the end of the time period applied for mixing during step (c) as it is without further treatment. It is, however, also possible within an alternative embodiment to practice a solid-liquid-separation according to step (e) of the inventive process. The solid-liquid-separation according to step (e) is carried out by any measure known to a person skilled in the art as suitable for the inventive purpose such as but not limited to filtration, pressing, membrane separation, flotation, precipitation, decantation and centrifugation or combinations thereof. Preferred are filter-based solid-liquid separations. It is further particularly preferred to use a filter press. The residues after the filtration should have a minimal solid content of 20 % (wt./wt.), preferably 25 % (wt./wt.), particularly preferred 30 % (wt./wt.) and most preferred 40 % (wt./wt.) solid content. Another method for the separation according to step (e) is centrifugation by e.g. using a decanter. In case the process according to the present invention involves solid-liquid-separation, the whole broth enzyme composition obtained according to step (d) of the inventive process is considered to be the liquid fraction.

Within a preferred embodiment of the inventive process, the process further comprises step
(aii) sterilization of the fermentation medium according to step (a) or the concentrated fermentation medium according to step (ai).

Sterilization can thereby be carried out by any means or measure known to a person skilled in the art as suitable for the inventive purpose. Within a preferred embodiment, sterilization is carried out by filtration, such as but not limited to membrane filtration processes or by ultra high temperature heating. A combination of two or more sterilization methods is also possible, however, it is particularly preferred to only apply ultra high temperature heating (also referred to as UHT). The UHT treatment is preferably carried out at a temperature of from 100 to 155 °C and for a duration of from 10 to 30 seconds, more preferred at a temperature of from 120 to 140 °C for a duration of from 10 to 20 seconds.

Within another aspect, the present invention relates to a filamentous fungus cell wherein SEQ ID NO:1 has been disrupted. Disruption of SEQ ID NO:1 can be carried out by any means and measure known to a person skilled in the art to be suitable for the inventive purpose. Possible and preferred methods and measures have been defined within the description. Within a preferred embodiment, SEQ ID NO:1 has been disrupted by deletion, mutation, modification of a promotor or any other regulatory sequence, generation of a stop codon or RNA interference. The term "filamentous fungus cell" has been defined within the description. All definitions given apply.

Within a preferred embodiment, the filamentous fungus cell is a genetically modified filamentous fungus cell with the ability to express at least one heterologous hydrolase enzyme. Within a particularly preferred embodiment, the filamentous fungus cell contains at least one heterologous beta glucosidase enzyme encoding sequence, at least one heterologous beta-xylosidase enzyme encoding sequence, at least one heterologous xylanase enzyme encoding sequence, at least one heterologous pectinase enzyme encoding sequence, at least one heterologous lytic polysaccharide monooxygenase enzyme encoding sequence, at least one heterologous oxidative enzyme encoding sequence and/or at least one heterologous accessory protein encoding sequence. The respective heterologous enzyme sequence may originate from any fungus or microorganism known to a person skilled in the art as suitable for the inventive purpose. Within a preferred embodiment the heterologous enzyme sequence originates from another species of filamentous fungus.

In another aspect the present invention relates to a whole broth enzyme composition prepared according to the process as defined before.

In a further aspect the present invention relates to the use of a whole broth enzyme composition as defined before for the hydrolyzation of lignocellulosic biomass. The definition within the description for "hydrolyzation" and "lignocellulosic biomass" apply.

### Generally preferred embodiments

In the following, generally preferred embodiments of the present invention are listed which do not limit the scope of the invention and/or scope of the claims in any respect. The generally preferred embodiments illustrate particularly suitable embodiments for the production of whole broth enzyme composition by the filamentous fungus *Trichoderma reesei.*

### Generally preferred embodiment 1

Process for production of a whole broth enzyme composition, comprising the following steps:
(a) providing a fermentation medium, originating from hydrolysis of lignocellulosic biomass, with a glucose content of from 40 to 400 g/L, a xylose content of from 50 to 200 g/L, a density of from 1.05 to 1.35 kg/L and a dry matter content of from 30 to 65 wt.-%;
(b) addition of at least one *Trichoderma reesei* cell wherein SEQ ID NO:1 has been disrupted by deletion, mutation, modification of a promotor or any other regulatory sequence, generation of a stop codon or RNA interference;
(c) mixing of the fermentation medium and the at least one *Trichoderma reesei* cell for a time period of from 1 min to 10 days at a temperature of from 20 to 35 °C;
(d) obtaining a whole broth enzyme composition.

### Generally preferred embodiment 2

Process for production of a whole broth enzyme composition, comprising the following steps:
(a) providing a fermentation medium, originating from hydrolysis of lignocellulosic biomass selected from wheat straw, barley straw, oat straw, rice straw, rye straw, bagasse or corn stover; with a glucose content of from 40 to 400 g/L, a xylose content of from 50 to 200 g/L, a density of from 1.05 to 1.35 kg/L, a dry matter content of from 30 to 65 wt.-%, a nitrogen content of from 0.05 to 5 wt.-%, a MgSO₄ content of from 200 to 350 mg/L and a average dissolved oxygen level of from 5 to 30%;
(b) addition of at least one *Trichoderma reesei* cell wherein SEQ ID NO:1 has been disrupted by deletion, mutation, modification of a promotor or any other regulatory sequence, generation of a stop codon or RNA interference;
(c) mixing of the fermentation medium and the at least one *Trichoderma reesei* cell for a time period of from 1 min to 10 days at a temperature of from 20 to 35 °C;
(d) obtaining a whole broth enzyme composition.

### Generally preferred embodiment 3

Process for production of a whole broth enzyme composition, comprising the following steps:
(a) providing a fermentation medium, originating from hydrolysis of lignocellulosic biomass selected from wheat straw, barley straw, oat straw, rice straw, rye straw, bagasse or corn stover; with a glucose content of from 40 to 400 g/L, a xylose content of from 50 to 200 g/L, a density of from 1.05 to 1.35 kg/L, a dry matter content of from 30 to 65 wt.-%, a nitrogen content of from 0.05 to 5 wt.-%, a MgSO₄ content of from 200 to 350 mg/L, a average dissolved oxygen level of from 5 to 30% and a pH of from 3.5 to 5;
(b) addition of at least one *Trichoderma reesei* cell wherein SEQ ID NO:1 has been disrupted by deletion, mutation, modification of a promotor or any other regulatory sequence, generation of a stop codon or RNA interference;
(c) mixing of the fermentation medium and the at least one *Trichoderma reesei* cell for a time period of from 1 min to 10 days at a temperature of from 20 to 35 °C;
(d) obtaining a whole broth enzyme composition containing at least one enzyme belonging to the class of cellulases and at least one enzyme belonging to the class of hemicellulases which has been produced by the at least one *Trichoderma reesei* cell.

### Generally preferred embodiment 4

Process for production of a whole broth enzyme composition, comprising the following steps:
(a) providing a fermentation medium, originating from hydrolysis of lignocellulosic biomass, with a glucose content of from 5 to 25 g/L, a xylose content of from 2 to 15 g/L, a density of from 1.05 to 1.35 kg/L and a dry matter content of from 30 to 65 wt.-%;
(b) addition of at least one *Trichoderma reesei* cell wherein SEQ ID NO:1 has been disrupted by deletion, mutation, modification of a promotor or any other regulatory sequence, generation of a stop codon or RNA interference;
(c) mixing of the fermentation medium and the at least one *Trichoderma reesei* cell for a time period of from 1 min to 10 days at a temperature of from 20 to 35 °C;
(d) obtaining a whole broth enzyme composition.

### Generally preferred embodiment 5

Process for production of a whole broth enzyme composition, comprising the following steps:
(a) providing a fermentation medium, originating from hydrolysis of lignocellulosic biomass selected from wheat straw, barley straw, oat straw, rice straw, rye straw, bagasse or corn stover; with a glucose content of from 5 to 25 g/L, a xylose content of from 2 to 15 g/L, a density of from 1.05 to 1.35 kg/L, a dry matter content of from 30 to 65 wt.-%, a nitrogen content of from 0.05 to 5 wt.-%, a MgSO₄ content of from 200 to 350 mg/L and a average dissolved oxygen level of from 5 to 30%;
(b) addition of at least one *Trichoderma reesei* cell wherein SEQ ID NO:1 has been disrupted by deletion, mutation, modification of a promotor or any other regulatory sequence, generation of a stop codon or RNA interference;
(c) mixing of the fermentation medium and the at least one *Trichoderma reesei* cell for a time period of from 1 min to 10 days at a temperature of from 20 to 35 °C;
(d) obtaining a whole broth enzyme composition.

### Generally preferred embodiment 6

Process for production of a whole broth enzyme composition, comprising the following steps:
(a) providing a fermentation medium, originating from hydrolysis of lignocellulosic biomass selected from wheat straw, barley straw, oat straw, rice straw, rye straw, bagasse or corn stover; with a glucose content of from 5 to 25 g/L, a xylose content of from 2 to 15 g/L, a density of from 1.05 to 1.35 kg/L, a dry matter content of from 30 to 65 wt.-%, a nitrogen content of from 0.05 to 5 wt.-%, a MgSO4 content of from 200 to 350 mg/L, a average dissolved oxygen level of from 5 to 30% and a pH of from 3.5 to 5;
(b) addition of at least one *Trichoderma reesei* cell wherein SEQ ID NO:1 has been disrupted by deletion, mutation, modification of a promotor or any other regulatory sequence, generation of a stop codon or RNA interference;
(c) mixing of the fermentation medium and the at least one *Trichoderma reesei* cell for a time period of from 1 min to 10 days at a temperature of from 20 to 35 °C;
(d) obtaining a whole broth enzyme composition containing at least one enzyme belonging to the class of cellulases and at least one enzyme belonging to the class of hemicellulases which has been produced by the at least one *Trichoderma reesei* cell.

### Generally preferred embodiment 7

Process for production of a whole broth enzyme composition as defined by any of generally preferred embodiments 1 to 6, wherein the *Trichoderma reesei* cell is further genetically modified by genetic methods (e.g. transformation) and/or non-genetic methods e.g. chemical mutagenesis or irradiation and wherein this further genetically modified *Trichoderma reesei* cell is able to express at least one heterologous hydrolase enzyme, at least one heterologous pectinase enzyme, at least one heterologous oxidative enzyme and/or at least one heterologous accessory protein.

### Generally preferred embodiment 8

*Trichoderma reesei* cell wherein SEQ ID NO:1 has been disrupted by deletion, mutation, modification of a promotor or any other regulatory sequence, generation of a stop codon or RNA interference and wherein the *Trichoderma reesei* cell is a genetically modified *Trichoderma reesei* cell, wherein the *Trichoderma reesei* cell comprises at least one heterologous beta glucosidase enzyme encoding sequence, at least one heterologous beta-xylosidase enzyme encoding sequence, at least one heterologous xylanase enzyme encoding sequence, at least one heterologous pectinase enzyme encoding sequence, at least one heterologous lytic polysaccharide monooxygenase enzyme encoding sequence, at least one heterologous oxidative enzyme encoding sequence and/or at least one heterologous accessory protein encoding sequence.

### Generally preferred embodiment 9

*Trichoderma reesei* cell as defined by generally preferred embodiment 8, wherein the at least one heterologous beta-glucosidase enzyme encoding sequence originates from *Cladophialophora* species, *Pseudocercospora* species and/or *Talaromyces* species and wherein the at least one xylanase enzyme encoding sequence originate from *Fomes* species, wherein the at least one beta-xylosidase encoding enzyme sequence originates from *Aspergillus* species and wherein the at least one lytic polysaccharide monooxygenase enzyme encoding sequence originates from *Aspergillus* species, *Trichoderma* species or *Hypocrea* species.

### Generally preferred embodiment 10

Whole broth enzyme composition produced according to a process as defined by any of generally preferred embodiments 1 to 7 containing at least one heterologous betaglucosidase enzyme produced by the genetically modified *Trichoderma reesei* cell as defined by generally preferred embodiment 8 or 9 and containing whole of or part of these *Trichoderma reesei* cell.

### Generally preferred embodiment 11

Use of a genetically modified Trichoderma reesei cell as defined by generally preferred embodiment 8 or 9 for the production of whole broth enzyme composition as defined by generally preferred embodiment 10 for the hydrolyzation of lignocellulosic biomass.

### Figures and examples

The present invention is described by the following figures and examples. It is thereby emphasized that the figures and examples do not limit the scope of the invention and claims but merely constitute further illustration of the invention, inventive purpose and benefits achieved by the inventive method.

### List of figures

- Figure 1:: Protein concentrations in the culture supernatants of pSEQ1M-HygR transformants MSEQ1-1 to -3 and reference strain M18.2b. Values are given in relation to the average protein concentration in the supernatants of the host strain M18.2b which is set to 1.
- Figure 2:: Biomass concentrations in the culture supernatants of pSEQ1M-HygR transformants MSEQ1-1 to -3 and reference strain M18.2b. Values are given in relation to the average biomass concentration in the supernatant of the host strain M18.2b which is set to 1.
- Figure 3:: Viscosity of culture broths of pSEQ1M-HygR transformants DSEQ1-1 and -3 and reference strain M18.2b. Values are given in relation to the viscosity of the culture broth of the host strain M18.2b which is set to 1.

### General

The examples describe the inactivation of the *Trichoderma reesei* SEQ ID NO:1 gene by mutation of the start codon and show the effect of the SEQ ID NO:1 gene inactivation on protein production, biomass formation and culture broth viscosity of T. *reesei.*

Standard methods known to those skilled in the art and described e.g. by Sambrook and Russel (Molecular Cloning - A laboratory manual; Cold Spring Harbor Laboratory Press, New York) or by Jansohn et al. (Gentechnische Methoden, Elsevier, München) were used for DNA agarose gel electrophorese, purification of DNA, transformation of *Escherichia coli,* plasmid propagation and purification, amplification of pieces of DNA by polymerase chain reaction (PCR) and isolation of genomic DNA from *Trichoderma reesei.* Ligation-independent cloning (LIC) was done essentially as described by Aslanidis and de Jong (1990, Nucleic Acid Res. 18 (20), 6069).

### Example 1: Construction of a SEQ ID NO: 1 mutation vector

Plasmid pSEQ1M (SEQ ID NO: 2) that contains the flanking regions for introduction of the mutations A1T and T2A (position according to SEQ ID NO: 1) into the SEQ ID NO:1 gene and a LIC site for insertion of the marker gene cloned into a pUC19-derived plasmid was synthesized by Thermo Fisher Scientific.

Plasmid pSEQ1M was digested with *Srf*I (New England Biolabs) according to the manufacturer's instructions and purified using the Wizard PCR purification kit from Promega.

The hygromycin B resistance cassette (HygR) (SEQ ID NO: 3) was synthesized by Thermo Scientific. HygR was amplified by PCR using the DNA from Thermo Scientific as template, primers SEQ1MHygRfw (5'- AACAAGACACAGCCCTATAAC -3'; SEQ ID NO: 4) and SEQ1MHygRrv (5'- AACAGACAAGAGCCCTATAAC -3'; SEQ ID NO: 5) and phusion polymerase from Thermo Fisher Scientific according to the manufacturer's instructions (annealing temperature: 68.5 °C, elongation time: 40 sec, 30 cycles). The amplicon (2.4 kb) was purified using the Wizard PCR purification kit from Promega.

The PCR-amplified HygR marker was fused with linearized pSEQ1M using ligation independent cloning (LIC). The linearized vector was treated with T4 DNA polymerase in the presence of dTTP. The PCR-amplified HygR marker gene was treated with T4 DNA polymerase in the presence of dATP. T4 DNA polymerase treated vector and marker gene were mixed and annealed as described in the cited literature. The assay was then transformed in chemically competent *Escherichia coli* XL1-Blue cells (Agilent), plated on LB-Agar plates containing 100 mg·l⁻¹ ampicillin (LB-Amp) and incubated at 37 °C for 24 h. Colonies were picked from the agar plates using toothpicks, transferred into liquid LB-Amp medium and incubated at 37 °C for 24 h with shaking (250 RPM). Plasmid DNA was isolated and integration of the insert was verified by digestion with *Pme*I*.* Plasmid clones were verified by Sanger sequencing and one plasmid with correct sequence was designated pSEQ1M-HygR.

### Example 2: Transformation of the SEQ ID NO: 1 mutation vector into Trichoderma reesei

Vector pSEQ1M-HygR was digested with *Pme*I (New England Biolabs) according to the manufacturer's instructions and the mutation cassette (5.9 kb) was purified by agarose gel electrophoresis and with the Wizard PCR purification kit from Promega. *Trichoderma reesei* M18.2b (DSM 19984) was transformed with the digested vector essentially as described in Penttilä et al (1987) Gene 61: 155-164 or Gruber et al (1990) Curr Genet 18: 71-76. The transformants were selected on potato dextrose agar plates containing 100 mg·l⁻¹ of hygromycin and 1 M sorbitol and purified by singularisation. Conidia stocks of the purified strains were prepared by growing them on potato dextrose agar plates at 30 °C until the plates were covered with spores. The conidia were harvested with sterile sodium chloride (0.9 g·l⁻¹)-Triton X-100 (0.01 g·l⁻¹) solution, adjusted to OD₆₀₀ = 10, supplemented with 50 g·l⁻¹ of glycerol and stored at -80 °C.

Genomic DNA was isolated from the mycelium of the transformants and the host strain. The integration of the *SEQ1* mutation cassette at the intended locus was verified by PCR using phusion polymerase from Thermo Fisher Scientific according to the manufacturer's instructions, genomic DNA from the transformants as template and primers SEQ1MKOfw (5'- GAGACCAGATCATCAGTC -3'; SEQ ID NO: 6) and SEQ1MKOrv (5'- CTGTACCTAGCAATAATAGC -3'; SEQ ID NO: 7) (annealing temperature: 56.0 °C, elongation time: 1 min 25 sec, 30 cycles). A 2.6 kb band with primers SEQ1MKOfw and SEQ1MKOrv indicates the integration of the mutation cassette at the SEQ ID NO:1 locus. Genomic DNA from strain M18.2b was also tested as a control. In order to verify that the intended mutation had been inserted into the *SEQ1* ORF, the 2.6 kb amplicon from the PCR with primers SEQ1MKOfw and SEQ1MKOrv was purified using the Wizard PCR purification kit from Promega and sequenced using Primer MSeq-01 (5'- GGTTGCAGGCAGAAGTATTG -3'; SEQ ID NO: 8).

Three strains containing the mutation from pSEQ1M-HygR in the *SEQ1* ORF were named MSEQ1-1 to -3.

### Example 3: Growth of the SEQ ID NO: 1 mutation strains in shake flasks

The strains MSEQ1-1 to -3 and M18.2b were grown in shake flasks in hydrolysate medium 1. Hydrolysate medium 1 contains (g.l⁻¹):

| Name | Concentration [g/l] |
|---|---|
| Acetic acid | 0.34 |
| Calcium | 0.12 |
| Chloride, water soluble | 0.15 |
| Copper | 0.0001 |
| Fat (HCI soluble) | 0.001 |
| Furfural | 0.003 |
| Glucose | 6.5 |
| Glycerol | 0.009 |
| HMF | 0.006 |
| Iron | 0.004 |
| Magnesium | 0.048 |
| Manganese | 0.002 |
| Na-D/L-Lactat | 0.097 |
| Nitrogen, soluble | 0.85 |
| Phosphorus | 0.48 |
| Phthalate | 8.2 |
| Potassium | 3.2 |
| Sodium | 0.015 |
| Sulfur | 0.86 |
| Xylose | 3.6 |
| Zinc | 0.001 |

The medium was adjusted to pH 5.5 with HCI or NaOH and sterilized by autoclaving (20 min at 121 °C).

15 ml of the medium were distributed into 50 ml Erlenmeyer shake flasks under a sterile hood. Conidia stocks of strains MSEQ1-1 to -3 and M18.2b were thawed, 75 µl of the conidia suspensions were pipetted into the Erlenmeyer flasks with the medium under a sterile hood and the flasks were closed with rubber foam caps. Three flasks were inoculated per strain. The flasks were incubated at 30 °C with shaking (250 RPM) for 6 days. After 6 days, the cultures were poured into 15 ml tubes. Aliquots were removed, centrifuged (3220xg, 4 °C, 15 min) and the supernatants stored at 4 °C, while the remaining culture broth was used for determination of the biomass and viscosity (see below).

### Example 4: Characterization of the culture supernatants and broths: Protein concentration. Biomass. Viscosity

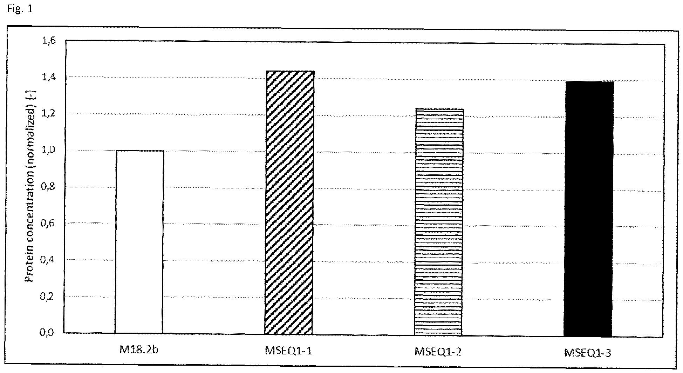

Protein concentrations in the centrifuged culture supernatants of strains MSEQ1-1 to -3 and M18.2b were measured using the Quick Start^{™} Bradford reagent (BioRad) and BSA standard solutions (BioRad) according to the supplier's instructions. The results of the measurements are shown in Figure 1 and are presented in relation to the average protein concentration in the culture supernatans of strain M18.2b, which is set to 1.

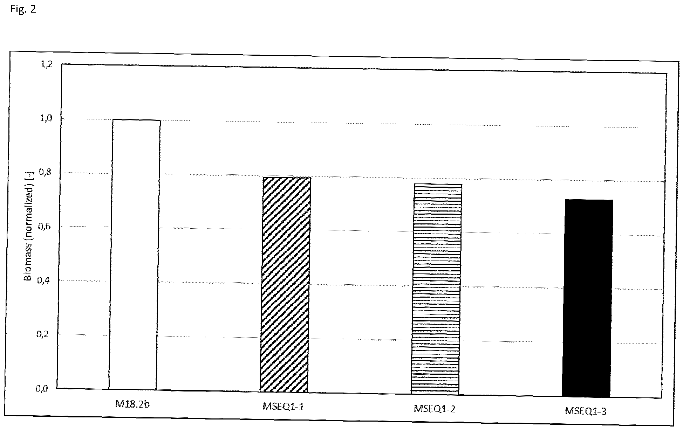

For biomass determination, Whatman^{™} filter discs (P1) were dried at 60 °C until their weight remained constant for 24 h. Culture broths of strains MSEQ1-1 to -3 and M18.2b were filtered using those dried filter discs and the mycelia were washed with at least ten times the broth's volume of deionized water. Then the filter discs with the mycelium were dried at 60 °C until their weight remained constant for 24 h. The filter discs with the dried mycelia were weighted. The biomass concentration in the culture broth was then calculated by subtracting the mass of the dried filter disc from the mass of the dried filter disc with the mycelia and then dividing that value by the volume of the culture broth that had been filtered. The results of the measurements are shown in Figure 2 and are presented in relation to the average biomass concentration in the culture broths of strain M18.2b, which is set to 1.

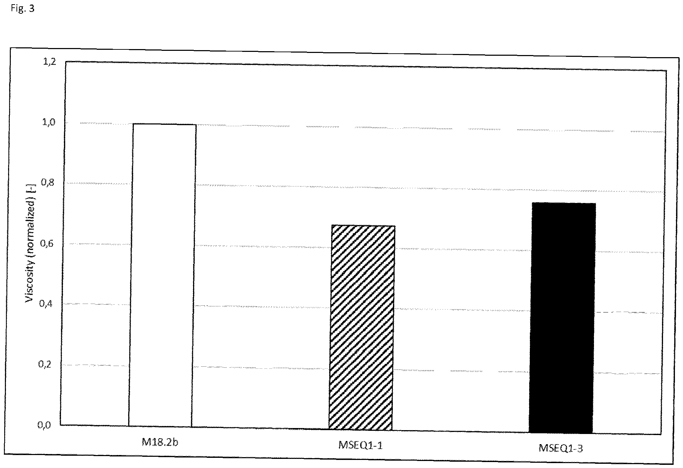

The viscosity of the culture broths of strains MSEQ1-1 and -3 and M18.2b was measured using a Malvern Kinexus Lab+ KNX2110 rotational rheometer with the Vane tool (4Vnn:CUPnn) according to the manufacturer's instructions. The measurements were taken at a temperature of 20 °C and at a rotation velocity of 18.11 RPM ("rotations per minute"). The viscosities are depicted in Figure 3 and are presented in relation to the viscosity of the culture broth of strain M18.2b, which is set to 1.

### Summary:

A person skilled in the art will recognize that inactivation of SEQ ID NO:1 results in a significant reduction of culture broth viscosity as well as a reduction of biomass formation while protein concentration is significantly increased

### Sequence listing

SEQ ID NO: 1
   SEQ1 native gene 
ATGATAGCCACAAGGTATCTGCTGAGAATCAGCCTGCTTCTCGTGGGCTTATTT
GCGTCACATGCCCTATCGAGCACCATCAATGCCGATCAGGAGTGTGTTTCTGCT
GTCTTTAGTATTGTGGGAGGTCTCAGCTTTGTAGGCGTCGGCTATGGCAAGTAC
TACGTAGCCAACTGCCAGAACCCGCTCAAAGTTGCCTCCATCTACGCCGTCTCC
AAGACCTACTGCCCGCCCTCTGACCTGGATCCTGGCATCGAATACGTCAAGTCG
TTATGTCGAAAGTACGGCGGCGCGGAACCTTTATCGGAGGCAGATGTGGCTGC
CAACCTGACGGATGAAGCAATCAGCCAGTATCCCATTCTTGATCAGACTGATGA
TCTGGTCTCGACGAATCTTACAACACCGGTTTTGATCTCGAAGCATTGGTTTGAT
CTGGGCTTCAAGACGGAGGTAAGTTTTCTTGGCCTCATTTCTCGTTGTGTAACA
CCGTTGCTAAGATTCTCTAGGTCGAGTGGGATGCTGCGGCTCGAAATCGCGAA
AACTATGGGTGAGATTACCCCCTCTGTACTGCTTGAAGAAAGCAAGGAGCTGAC
ATGTATAACACTACAGATGGGCCATGTACGGATTCTGGGGCATCGTTCTCCTCT
TCGGAACTTTTCATCGCCTCATCTCCCACTTCCTCGAAGGCCGTGTGCTTCCGT
 
CGTCGACCGGCCCCGAAGATACCGGATCGGCTGATAGCCGTGAAAATCCAGA
GCAGGAACCAAGTCACTTGGGCATCTGTACTCGAGAATACGCAAGGGCCTTACT
ACTCCCAGTGTTTTCCCTCCGTATCACCAGCAGCTTCTGTTTGGGTGCACGATC
CCTACTCGGCTTGAGCTATTCATCGTTGCCTCGTACTGGATCGTCAGCTTCGTG
CTCTGCTGCGTCAGCTATCGTCCATTCGACGGCAACCTATAGTAAGTAGCCATA
CTGAAAAAGATGAACAAACTCCTTACGAGATACAAACGACTAACAGAGGCTTACT
GGAAGCTGGCCCAAGGTTTCTGCTCAGCTATGGCGATATATTGCCGACCGAAC
GGGCGTCATGTCGTACGCCAACCTACCGCTGATATGGATGTTTAGCGGCCGAA
ATAACATCTTCATCTGGCTGACGGGCTGGCAATTCTCCACGTTCAACCTGTTCC
ACAGACACATTGCTCGCGTGGCGACATTGCAGGCCATTATTCACTCCGTTGGAT
ACACGGCATTTTACTACACGGCCGGTTACGGTATGTTGATCGCTTGCCTCTCAT
GGAGAGTTTGCATTGAAGTTCTCCGTTGACAAGAGTAATTTCAGGGTATCAATTC
CCACCATTGTTCCGTGAGCCTTGGTTCTACCTCGGCGTTGTGGTAAGCCTTTTG
GCAGACATTTCTTATGGCCTGTTTGCTGATCATGACCCACTGTAGGCAACGTGT
GCGATGAGCTTTATTGTCTTCTTCTCCTTCAGCTGGTTCCGCAGAAAGATTTACG
AGTCATTCCTACTCATCCACATCGTCCTATCTGTCGTGGTGATTGTCGCCCTGTT
CTAGTACGTTCTCCTCCTGGTCCGGTGCTTCTCATTGTCCATCTCATACTGACTA
GACTTGGAATCACAGCCACACATCCATCTTTGACGGCCAATATGACCCATATCTA
TGGCCTCTCGTTGCCATCTGGAGCTTCGATCGTTTCCTGAGAATCGTCCGGGTA
GTCTACTGCAATATCCACGTTCGGCTGCGTAAGACAAAGCTCCAGAGAAGCATC
GCGGCCATAGCTTACGACCAGGACGCCAACATCATCACCGTCCAGATCAACAC
ACATGTGAAACCGGGACCAGGACAGCACTATTATCTGTACCAGCCCTTCCGCCT
CAAAGGCTGGGAGAATCACCCTTTCACCCTGGCCCATTGGAACCAGCCTGGAG
GACAAACAGGATCAACAGATGTGCAATCGAGAACCGTGGATGGCTCTGTTGTGA
CATCGTTCGAGAGCGGTTCGCCACATGGAGGCATGGCAGGCGAGCATCTTCTT
TCCTTCTGGATTCGCCCCTATGACGGCTGGACTCGCCATCTCCGTGACGTCTGT
CTCAAGGCTCAGACCAAGTCCCAGGTTCCCGCAGTCCATAAAGAGACCATCCTC
CTCGAAGGCCCCTACGGCATGGCGGAGCCCTTGTGGGCTTATGACGAGGTCCT
TCTCTTCGCCGGCGGGTCTGGTATCGCGACCATGGTTCCGTACATACTTGATCA
CATGGAAAGAACCACTGCGTCTAACAAAGCGAGACGAACACGCACACGCGCAT
TGACGCTCGTGTGGTCAAATCGCAGCCAAGCTTTCATGAAGCGGGTTGCCCAG
CGCGAGCTCGCCGCGGCACTTGCGCGAGACGACATCGAGTGTATGTTTCACTG
CACAAGCACTGCAAAGGAGTCGCTTGACTCGCTGCCCTTGACGCCGAAGGATG
AGATTTCGGCGGAGAAGGAGTGTAAAGGGATGGCGGAGAGCAGTGTGCTCAG
 
GGGCAAGGAGGAGACGGATGTGTCTGAGTCGCCTGATGGTGCGGCTGTGGCA
ATTAGGATGGGCAGGCCAGATATCGGGGTCATGATTGAGAAAGCGGCTGAGTC
TGCCTCTGCGTCTGGATCACGGCTGGCGGTGTTTGCGTGTGGCCCTGGAGAGA
TGGCGGATGCGGCGAGGGCGGCGACATATCGGGCGATGAAGCAGAGCCATTC
TGTCGAGTATTTCGAGGAGGCGTTTGGGTGGTAA
SEQ ID NO: 2
   pSEQ1M 
CTAAATTGTAAGCGTTAATATTTTGTTAAAATTCGCGTTAAATTTTTGTTAAATCAG
CTCATTTTTTAACCAATAGGCCGAAATCGGCAAAATCCCTTATAAATCAAAAGAA
TAGACCGAGATAGGGTTGAGTGGCCGCTACAGGGCGCTCCCATTCGCCATTCA
GGCTGCGCAACTGTTGGGAAGGGCGTTTCGGTGCGGGCCTCTTCGCTATTACG
CCAGCTGGCGAAAGGGGGATGTGCTGCAAGGCGATTAAGTTGGGTAACGCCAG
GGTTTTCCCAGTCACGACGTTGTAAAACGACGGCCAGTGAGCGCGACGTAATA
CGACTCACTATAGGGCGAATTGGCGGAAGGCCGTCAAGGCCGCATGACTGTTT
AAACACACTCCTGATCGGCATTGATGGTGCTCGATAGGGCATGTGACGCAAATA
AGCCCACGAGAAGCAGGCTGATTCTCAGCAGATACCTTGTGGCTATCGAGGCT
GGATGATCTTCAGGTCCTGGGAAGTCGACTGTCCCACAAACAGATAAGAACTTT
CCACGGCACGGACGCCAGATATACATCAGCTCGGCAACTTGCGTTATATAACT
ACTCATTCCTACGATCCCTCGGCAGCTCGTGAACCGTACAACGGTGTCCGCTCA
CGCCACTGTGGGCTTTTCAAAAACCAATACTTCTGCCTGCAACCGGGGAATCTT
GATCCCACTCAGCCCGTATCCATCACCACTCGGATTCCGTCCCCCAGGTAGTTG
GCACGCCCTTCATCAACACTCGCAGGATCAAGGTTCTATACCCGCAATATAAAG
AAAAAAAACTTCGTCAAGGTGGAGGCACGTTGCTCGACATGGGCTTGATCAGCG
GTCTAGATCAGTTGATGCATGTCCTAGGGTCGTATCTGACTCAATCCTTTAGGTA
AAGGTGGACTGATCAGAAATCTTATCAGATTGATATTGACTCATCAGATTAGATT
AGATCTGATCCTGCCGTGCGAGTGGCTCTGTCGAGGAGAAAGCCCACTTAATCT
TCTCCCTTGTCTTGCCTGATTAGGAACGGTCCTTTGTCTCTTCTCCGGCTTCCTT
ATGCGACATGCCCTCCGGGAATAGGCTCAAGCAGTTGAGCCATTTAATTGCCTA
TACTGGTTTTTTTTGGCTTGTGACGCTCCGGTCTTTTCTCTCGGATTGTGTCGAG
ATCAAGATTTTCCAGCAATATATTGGCAGATGTGGATTGACTGGGGAGCAACTG
ACTGCCGCTGCAGGCCTTGAGATCCCGGTTTCCGCAAACTTTACATCCGAAGTT
CCTCTTCGAAGTGAAGACAAAGAGACCCAGATTTTGCCCAAAGGCCCGATATTC
CAGCAGTCCGTATTGCGGCTGAGGGTGGATCATCCTTGAAGTGGCGACAGAGC
 
AAGCTTGTTTTTACTCTTCTTTATCTCGCCATGGATAGTCCATCGGATACGGAC
ACTGGTGCTGCTTCGGAGATATACTTGTTCAGTCTCACAACGCTACAATGCTATG
GCTACTCAAGTGTTCCAGAGAGGATTATTGTCAATATGCGACTTCTCCTGATCCC
ACAATGTTTGGATCTTCACATATTGATGCTGATGATCTGTGGGCTTTCCTTATAG
CCTCTATTCCACTAGATGTCCTACTATATCGGTAATCTAGGATTTACAGGATTTC
TCAGAAATAGGTGGCTCAGCCGTGACTCGAGACTATGGATACTACCTACTTCAA
GACCAACTATGCACCAAGAGAGGACAGAATAACATACCTAAGTAAGAGCCATAG
GAAGTGGGAAACAGCCACAGTACTTTGTACTATCTCTAGAGCAAATCAGTCGCC
GTGCATCCTAGTCACCGTATACCACTGCAGACTCTAATCTCTATCTTAACCATAA
GTATCCTGGTGTTTCACACAGCAAGGGTAGCAGTACAAAAGTACCGTCACACAG
GCCAAGACTTGGCATAGTGGGCAGAGGCAGCGGTCCGGGTAAGCAGCTCCGT
CTTCGGGTCATCGAGTTAGTAGGCTATGGCCGGGACATCCATCGAATGAAGATA
CGAATTGCCAAATAGGATCTATGAGAGTTGCCATAAGCCATCCGATTATAAATAA
GAGATCACCAGCCGAGACGCTAGGCTTAAGAAGAGTAGCCTCGCCAGCAGAAT
AGCATAGGAATATGTTGTATCTTTAGTGCACTAGCAAAAAGAGTCATAGCAACTG
GCGTTGTAGTTACACTCCAAGTAAGAATAATGGTTCTCGAATACATGTAGCCTTG
ATGAGACTATTCTGGACGCTGTTACCCATGTGTGCCTTGCATATATCTCAGTATG
AGAGTTTAAGATCCCGGCAATGCAGAAGCGAAGTTGCTCAGCTTCCTAACAAGA
CACAGCCCGGGCTCTTGTCTGTTAACTGCCTGCATGCAGAAAGGACGCACTT
CTTCCCACCTACAAGAGACTGGAGAAATGGAGTGATATATCACTGGACAGACGA
GGGAAAAGAGCTCGACTTCACGATCAAAGGGCATTACGACTCACTTCCTTCGCT
AATCAGACGAACCCCTACTTATTAAGGGCAACATTCACACTCTAAACGATTAATA
ACACCAAAGTCTGCCATTATCTACAGATTATGCTCCATTCCTAGTATATATAACCA
TAGGTAGAGGAGAGCTCGGGGTCCTGTTAAGCACTTCTCTTAAGTATATTATCT
GCTATTTTTAATAGCTAGGGGATCTAAATCTAGGGTTATATACTTCTATTTCTAGT
AGTTAACTAACCCTATATTAAGGTAGTTACTACTATTTTACTTTAATTAAACATGCT
ACTAATAGTACTAGTAAAACTAGGCTAAGTCTATTTTTTACTAAGCCCTTAAATAT
TACCTTAATAATTTCACCCCCTTTTACTTAAGACTAGGTTATTTTTAATACTAGTAC
TGCTACTACTTACTTTATATAAGGTAAAGTTATATATAATACTACACCTTCCCTGC
ACTTTATATAATATTCTTATATTACTTATTAGCGCTTATTTTCTCTCTTTTTATAACT
CTTTTATCTTATTAATGCATATCCTTAATAACTATAATAACTAATTTTATAATAATTA
CTACTACTACCTTTATAGCTATAATTATAACTATATATTAAAAGCTAAGTAAGGAT
ATATAATATATTTATATATATTCTATACCTTACTACTTACTTATAGTTTAACTACTTT
ATAAAACTAAACTATTAAACTTAATATATAAAGCTATAATATTTTTTTTAAATAGTAT
 
TAAAACTATTTTAATACTTTTCTAATCCTTATTCTTTATTATAAGTAATATTTATTTA
AGGTAACTAACTTTATAAAGTATATAAAAAGTAAGGAAGAATTTAAGTAACTTTTT
AAAAAAGAGAATAAAAAAAAGCTTAAGAAATACTATAACTTAATATAAAAAGTATA
TTTAAAGATAATTTATAACTATAAGATCTTTTTATATAAAAATGCTAAGAATATAGC
AGTAAGGATATACTATAGTACCTTTAAGTCTTTTATATACTTTTTAAAGGGCACTA
TTTTTAACTAGGAAGCTAATAAAGTTAAGAGCTTAGCACTTAATATAAATATTCTA
GTTTAAAAAGGGGATAATATTTACTTAAGTTCTTATAAGGACTATAGGTTAATATA
ATTACTTTTTTATTAAAACTGTAACTAAAAGCTAAATTATTATAGTAACTGCAGACT
ATAACTAAATATTTATAAGTATTATTTAATAATATAACCTAAGGATATTAATATTAA
CCCTATATAATATATAAATACTGTTTATTATAATTAATAAATAAAGCCTATAGTTAA
TAGTATTATAACTAGTAAAGATAAGCTAAAGACAGTTACTTTAAGAAAGCAGTTA
GTTTAAACGACTCTGGCCTCATGGCCTTCCGCTCACTGCCCGCTTTCCAGTC
GGGAAACCTGTCGTGCCAGCTGCATTAACATGGTCATAGCTGTTTCCTTGCGTA
TTGGGCGCTCTCCGCTTCCTCGCTCACTGACTCGCTGCGCTCGGTCGTTCGGG
TAAAGCCTGGGGTGCCTAATGAGCAAAAGGCCAGCAAAAGGCCAGGAACCGTA
AAAAGGCCGCGTTGCTGGCGTTTTTCCATAGGCTCCGCCCCCCTGACGAGCAT
CACAAAAATCGACGCTCAAGTCAGAGGTGGCGAAACCCGACAGGACTATAAAG
ATACCAGGCGTTTCCCCCTGGAAGCTCCCTCGTGCGCTCTCCTGTTCCGACCCT
GCCGCTTACCGGATACCTGTCCGCCTTTCTCCCTTCGGGAAGCGTGGCGCTTT
CTCATAGCTCACGCTGTAGGTATCTCAGTTCGGTGTAGGTCGTTCGCTCCAAGC
TGGGCTGTGTGCACGAACCCCCCGTTCAGCCCGACCGCTGCGCCTTATCCGGT
AACTATCGTCTTGAGTCCAACCCGGTAAGACACGACTTATCGCCACTGGCAGCA
GCCACTGGTAACAGGATTAGCAGAGCGAGGTATGTAGGCGGTGCTACAGAGTT
CTTGAAGTGGTGGCCTAACTACGGCTACACTAGAAGAACAGTATTTGGTATCTG
CGCTCTGCTGAAGCCAGTTACCTTCGGAAAAAGAGTTGGTAGCTCTTGATCCGG
CAAACAAACCACCGCTGGTAGCGGTGGTTTTTTTGTTTGCAAGCAGCAGATTAC
GCGCAGAAAAAAAGGATCTCAAGAAGATCCTTTGATCTTTTCTACGGGGTCTGA
CGCTCAGTGGAACGAAAACTCACGTTAAGGGATTTTGGTCATGAGATTATCAAA
AAGGATCTTCACCTAGATCCTTTTAAATTAAAAATGAAGTTTTAAATCAATCTAAA
GTATATATGAGTAAACTTGGTCTGACAGTTACCAATGCTTAATCAGTGAGGCACC
TATCTCAGCGATCTGTCTATTTCGTTCATCCATAGTTGCCTGACTCCCCGTCGTG
TAGATAACTACGATACGGGAGGGCTTACCATCTGGCCCCAGTGCTGCAATGATA
CCGCGAGAACCACGCTCACCGGCTCCAGATTTATCAGCAATAAACCAGCCAGC
CGGAAGGGCCGAGCGCAGAAGTGGTCCTGCAACTTTATCCGCCTCCATCCAGT
 
CTATTAATTGTTGCCGGGAAGCTAGAGTAAGTAGTTCGCCAGTTAATAGTTTGCG
CAACGTTGTTGCCATTGCTACAGGCATCGTGGTGTCACGCTCGTCGTTTGGTAT
GGCTTCATTCAGCTCCGGTTCCCAACGATCAAGGCGAGTTACATGATCCCCCAT
GTTGTGCAAAAAAGCGGTTAGCTCCTTCGGTCCTCCGATCGTTGTCAGAAGTAA
GTTGGCCGCAGTGTTATCACTCATGGTTATGGCAGCACTGCATAATTCTCTTACT
GTCATGCCATCCGTAAGATGCTTTTCTGTGACTGGTGAGTACTCAACCAAGTCAT
TCTGAGAATAGTGTATGCGGCGACCGAGTTGCTCTTGCCCGGCGTCAATACGG
GATAATACCGCGCCACATAGCAGAACTTTAAAAGTGCTCATCATTGGAAAACGTT
CTTCGGGGCGAAAACTCTCAAGGATCTTACCGCTGTTGAGATCCAGTTCGATGT
AACCCACTCGTGCACCCAACTGATCTTCAGCATCTTTTACTTTCACCAGCGTTTC
TGGGTGAGCAAAAACAGGAAGGCAAAATGCCGCAAAAAAGGGAATAAGGGCGA
CACGGAAATGTTGAATACTCATACTCTTCCTTTTTCAATATTATTGAAGCATTTAT
CAGGGTTATTGTCTCATGAGCGGATACATATTTGAATGTATTTAGAAAAATAAAC
AAATAGGGGTTCCGCGCACATTTCCCCGAAAAGTGCCAC
SEQ ID NO: 3
   Hygromycin B resistance marker 
GTTAACAAGACACAGCCCTATAACTTCGTATAATGTATGCTATACGAAGTTATAT
AACGGTGAGACTAGCGGCCGGTCCCCTTATCCCAGCTGTTCCACGTTGGCCTG
CCCCTCAGTTAGCGCTCAACTCAATGCCCCTCACTGGCGAGGCGAGGGCAAGG
ATGGAGGGGCAGCATCGCCTGAGTTGGAGCAAAGCGGCCCGGCCGCCATGGG
AGCAGCGAACCAACGGAGGGATGCCGTGCTTTGTCGTGGCTGCTGTGGCCAAT
CCGGGCCCTTGGTTGGCTCACAGAGCGTTGCTGTGAGACCATGAGCTATTATTG
CTAGGTACAGTATAGAGAGAGGAGAGAGAGAGAGAGAGAGAGAGGGGAAAA
AAGGTGAGGTTGAAGTGAGAAAAAAAAAAAAAAAAAAAAATCCAACCACTGACG
GCTGCCGGCTCTGCCACCCCCCTCCCTCCACCCCAGACCACCTGCACACTCAG
CGCGCAGCATCACCTAATCTTGGCTCGCCTTCCCGCAGCTCAGGTTGTTTTTT
TTTCTCTCTCCCTCGTCGAAGCCGCCCTTGTTCCCTTATTTATTTCCCTCTCCAT
CCTTGTCTGCCTTTGGTCCATCTGCCCCTTTGTCTGCATCTCTTTTGCACGCATC
GCCTTATCGTCGTCTCTTTTTTCACTCACGGGAGCTTGACGAAGACCTGACTCG
TGAGCCTCACCTGCTGATTTCTCTCCCCCCCTCCCGACCGGCTTGACTTTTGTT
TCTCCTCCAGTACCTTATCGCGAAGCCGGAAGAACCTCTTAACCTCTAGATGAA
AAAGCCTGAACTCACCGCCACGTCTGTCGAGAAGTTCCTGATCGAAAAGTTCGA
CAGCGTCTCCGACCTGATGCAGCTCTCGGAGGGCGAAGAATCTCGTGCTTTCA
 
GCTTCGATGTAGGAGGGCGTGGATATGTCCTGCGGGTAAATAGCTGCGCCGAT
GGTTTCTACAAAGATCGTTATGTTTATCGGCACTTTGCATCGGCCGCGCTCCCG
ATTCCGGAAGTGCTTGACATTGGGGAATTCAGCGAGAGCCTGACCTATTGCATC
TCCCGCCGTGCACAGGGTGTCACGTTGCAAGACCTGCCTGAAACCGAACTGCC
CGCTGTTCTGCAGCCGGTCGCGGAGGCCATGGATGCGATCGCTGCGGCCGAT
CTCAGCCAGACGAGCGGGTTCGGCCCATTCGGACCGCAAGGAATCGGTCAATA
CACTACATGGCGTGATTTCATATGCGCGATTGCTGATCCCCATGTGTATCACTG
GCAAACTGTGATGGACGACACCGTCAGTGCGTCCGTCGCGCAGGCTCTCGATG
AGCTGATGCTTTGGGCCGAGGACTGCCCCGAAGTCCGGCACCTCGTGCACGC
GGATTTCGGCTCCAACAATGTCCTGACGGACAATGGCCGCATAACAGCGGTCAT
TGACTGGAGCGAGGCGATGTTCGGGGATTCCCAATACGAGGTCGCCAACATCT
TCTTCTGGAGGCCGTGGTTGGCTTGTATGGAGCAGCAGACGCGCTACTTCGAG
CGGAGGCACCCGGAGCTTGCAGGATCGCCGCGGCTCCGGGCGTATATGCTCC
GCATTGGTCTTGACCAACTCTATCAGAGCTTGGTTGACGGCAATTTCGATGATG
CAGCTTGGGCGCAGGGTCGATGCGACGCAATCGTCCGATCCGGAGCCGGGAC
TGTCGGGCGTACACAAATCGCCCGCAGAAGCGCGGCCGTCTGGACCGATGGC
TGTGTAGAAGTACTCGCCGATAGTGGAAACCGACGCCCCAGCACTCGTCCGAG
GGCAAAGGAATAGATGCATGGCTTTCGTGACCGGGCTTCAAACAATGATGTGCG
ATGGTGTGGTTCCCGGTTGGCGGAGTCTTTGTCTACTTTGGTTGTCTGTCGCAG
GTCGGTAGACCGCAAATGAGCAACTGATGGATTGTTGCCAGCGATACTATAATT
CACATGGATGGTCTTTGTCGATCAGTAGCTAGTGAGAGAGAGAACATCTATC
CACAATGTCGAGTGTCTATTAGACATACTCCGAGAATAAAGTCAACTGTGTCTGT
GATCTAAAGATCGATTCGGCAGTCGAGTAGCGTATAACAACTCCGAGTACCAGC
GAAAGCACGTCGTGACAGGAGCAGGGCTTTGCCAACTGCGCAACCTTGCTTGA
ATGAGGATACACGGGGTGCAACATGGCTGTACTGATCCATCGCAACCAAAATTT
CTGTTTATAGATCAAGCTGGTAGATTCCAATTACTCCACCTCTTGCGCTTCTCCA
TGACATGTAAGTGCACGTGGAAACCATACCCAATATAACTTCGTATAATGTATGC
TATACGAAGTTATAGGGCTCTTGTCTGTTAAC
SEQ ID NO: 4
   SEQ1MHygRfw
   AACAAGACACAGCCCTATAAC
SEQ ID NO: 5
   SEQ1MHygRrv
   AACAGACAAGAGCCCTATAAC
SEQ ID NO: 6
   SEQ1MKOfw
   GAGACCAGATCATCAGTC
SEQ ID NO: 7
   SEQ1MKOrv
   CTGTACCTAGCAATAATAGC
SEQ ID NO: 8
   MSeq-01
   GGTTGCAGGCAGAAGTATTG

## Claims

1. Process for production of a whole broth enzyme composition, comprising the following steps:
(a) providing a fermentation medium, originating from hydrolysis of lignocellulosic biomass, with a glucose content of from 5 to 450 g/L, a xylose content of from 2 to 300 g/L, a density of from 1 to 2 kg/L and a dry matter content of from 10 to 75 wt.-%;
(b) addition of at least one filamentous fungus cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a whole broth enzyme composition.

2. Process according to claim 1, wherein the pH of the fermentation medium according to step (a) has been adjusted to a pH selected from pH 2.0 to 6.0.

3. Process according to any of the foregoing claims, wherein the ratio from glucose to xylose is from 1.0 to 3.5.

4. Process according to any of the foregoing claims, further comprising the step (ai) concentration of the fermentation medium by evaporation, membrane filtration or thin layer evaporation to decrease the weight of the fermentation medium by factor 2 to 6.

5. Process according to any of the foregoing claims, further comprising step (aii) sterilization of the fermentation medium according to step (a) or the concentrated fermentation medium according to step (ai).

6. Process according to any of the foregoing claims, wherein the fermentation medium according to step (a) has a furfural content of less than 0.5 g/L.

7. Process according to any of the foregoing claims, wherein the fermentation medium according to step (a) has a hydroxymethyl furfural (HMF) content of less than 0.5 g/L.

8. Process according to any of the foregoing claims further comprising the step
(e) solid-liquid separation of the fermented medium according to step (c) to obtain a solid fraction and a liquid fraction.

9. Process according to any of the foregoing claims wherein from 0.05 to 5 wt.-% nitrogen are added during step (a) and/or (b) of the process.

10. Process according to any of the foregoing claims wherein from 0.5 to 350 mg/L FeSO₄, MnSO₄, MgSO₄ and/or ZnSO₄ are added during step (a) and/or (b) of the process.

11. Process according to any of the foregoing claims, wherein the filamentous fungus cell is selected from the group consisting of *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes.*

12. Process according to any of the foregoing claims, wherein the filamentous fungus cell comprises at least one heterologous beta-glucosidase enzyme.

13. Filamentous fungus cell wherein SEQ ID NO:1 has been disrupted.

14. Filamentous fungus cell according to claim 13, wherein SEQ ID NO:1 has been disrupted by deletion, mutation, modification of a promotor or any other regulatory sequence, generation of a stop codon or RNA interference.

15. Filamentous fungus cell according to any of claims 13 or 14, wherein the at least one filamentous fungus cell is a genetically modified filamentous fungus cell with the ability to express at least one heterologous hydrolase enzyme, at least one heterologous pectinase enzyme, at least one heterologous oxidative enzyme and/or at least one heterologous accessory protein.

16. Filamentous fungus cell according to any of claims 13 to 15, wherein the at least one filamentous fungus cell is a genetically modified filamentous fungus cell comprising at least one heterologous beta glucosidase enzyme encoding sequence, at least one heterologous beta-xylosidase enzyme encoding sequence, at least one heterologous xylanase enzyme encoding sequence, at least one heterologous pectinase enzyme encoding sequence, at least one heterologous lytic polysaccharide monooxygenase enzyme encoding sequence, at least one heterologous oxidative enzyme encoding sequence and/or at least one heterologous accessory protein encoding sequence.

17. Whole broth enzyme composition produced according to a process as defined in any of claims 1 to 12.

18. Use of a filamentous fungus cell as defined in any of claims 13 to 16 for the production of whole broth enzyme composition.

19. Use of a whole broth enzyme composition according to claim 18 for the hydrolyzation of lignocellulosic biomass.
